# EUROPÄISCHE PATENTSCHRIFT

(11) **EP 1 827 301 B1**
(45) Veröffentlichungstag und Bekanntmachung des Hinweises auf die Patenterteilung: **12.07.2017**
(21) Anmeldenummer: 05791920.1
(22) Anmeldetag: 16.09.2005
(51) Int. Cl.: A61F 2/89, A61F 2/91, A61F 2/915

(54) **STÜTZPROTHESE**
SUPPORTING PROSTHESIS
PROTHESE DE RENFORT

(30) Priorität: 17.09.2004 DE 102004045224
(43) Veröffentlichungstag der Anmeldung: 05.09.2007
(73) Patentinhaber: Fliedner, Thilo, Dr., 81541 München (DE)
(72) Erfinder: Fliedner, Thilo, Dr., 81541 München (DE)
(74) Vertreter: Herrmann, Franz
(86) Internationale Anmeldenummer: PCT/DE2005/001633
(87) Internationale Veröffentlichungsnummer: WO 2006/029617

(56) Entgegenhaltungen:
- WO-A-01/21101
- WO-A-03/057075
- WO-A-03/057077
- DE-A1- 10 223 399

## Beschreibung

Die Erfindung betrifft eine Stützprothese für Gefäße oder intrakorporale Lumina mit einem rohrförmigen, in radiale Richtung expanierbaren Mantel, der eine metallische Stützstruktur mit quer zur Längsrichtung verlaufenden Stützelementen aufweist.

Eine derartige Stützprothese ist aus der DE 101 53 340 A1 bekannt. Die bekannte Stützprothese weist eine Vielzahl von Stützringen auf, die durch in Längsrichtung expandierbare Zwischenringe verbunden sind. Sowohl die Stützringe als auch die Zwischenringe weisen einen mäanderförmigen Verlauf auf. Derartige Stützprothesen sind dem Fachmann auch unter der Bezeichnung Stent bekannt. Die bekannte Stützprothese dient insbesondere der Behandlung von Gefäßverengungen oder so genannten Stenosen.

Die bekannte Stützprothese eignet sich insbesondere für die Implantation in stark gekrümmten Gefäßen, da sich die bekannte Stützprothese auch nach der Expansion an die Krümmung des zu stützenden Gefäßes anpasst. Außerdem ist die bekannte Stützprothese ausreichend flexibel, um einer Bewegung des Gefäßes, zum Beispiel einer Bewegung eines Herzkranzgefäßes, folgen zu können.

Ein Nachteil der bekannten Stützprothese ist, dass die Fertigung der Stützringe und der Zwischenringe sehr aufwendig ist. Sowohl die Stützringe als auch die Zwischenringe können grundsätzlich aus dünnen Drähten gebogen werden. Bei Durchmessern der Stents im Bereich von einem Millimeter ist dies nur mit unverhältnismäßig großem Aufwand möglich. Daneben besteht die Möglichkeit, die Stützringe und Zwischenringe beispielsweise mit Hilfe von Lasern aus Rohrmaterial herauszuarbeiten, auch wenn ein derartiges Herstellungsverfahren kostenintensiv und langwierig ist.
Aus der EP 0 997 115 A2 sind ferner Stents bekannt, die insbesondere der Behandlung von Gefäßaussackungen oder so genannten Aneurysmen dienen. Zum Einbringen der Stents kann der Durchmesser der bekannten Stents durch Falten der Stents verkleinert werden. Der bekannte Stent kann eine Vielzahl von Ringen oder eine Helix aufweisen, die an einem aus einem nichtmetallischen Material hergestellten Rohr angebracht sind.
Aus der EP 0 918 496 B1 sind außerdem ein Stent bekannt, der ebenfalls der Behandlung von Aneurysmen dient. Bei dem bekannten Stent ist ein in radiale Richtung expandierbarer rohrförmiger Einsatz von Halteringen gesichert, die untereinander durch Verbindungselemente verbunden sind, die eine axiale Relativbewegung der Halteringe untereinander zulassen. Als Material für die Verbindungselemente wird unter anderem auch ein Polymermaterial vorgeschlagen.

Die WO 01/21101 offenbart eine Stützprothese gemäß dem Oberbegriff von Anspruch 1.

Ausgehend von diesem Stand der Technik liegt der Erfindung daher die Aufgabe zugrunde, eine einfach herstellbare Stützprothese zur Behandlung von Verengungen zu schaffen.
Diese Aufgabe wird durch eine Stützprothese mit den Merkmalen des unabhängigen Anspruchs gelöst. In davon abhängigen Ansprüchen sind vorteilhafte Ausgestaltungen und Weiterbildungen angegeben.
Die Stützprothese für Gefäße oder intrakorporale Lumina weist einen rohrförmigen Mantel auf, der eine metallische Stützstruktur mit quer zur Längsrichtung verlaufenden Stützelementen aufweist. Die in Längsrichtung nebeneinander angeordneten Stützelemente sind durch wenigstens ein sich in Längsrichtung erstreckendes Verbindungselement aus einem elastischen nichtmetallischen Material zu dem röhrenförmigen Mantel verbunden.

Die Verbindungselemente lassen ferner bei der radialen Expansion eine Streckbewegung der Stützelemente zu und halten die Stützelemente bei der radialen Expansion auf Abstand.

Nichtmetallische Materialien können einen so hohen Elastizitätsmodul aufweisen, dass die Verbindungselemente nicht durch eine spezifische Formgebung als Federelemente ausgebildet werden müssen. Vielmehr können für die Verbindungselemente auch grobe Strukturen ausgewählt werden. Derartige grobe Strukturen können einfach gefertigt werden und sind in der Lange, die Stützelemente bei der radialen Expansion auf Abstand zu halten. In der Regel erfolgt die Befestigung der nichtmetallischen Verbindungselemente an den Stützelementen durch Adhäsion. Die Kontaktstellen zwischen den nichtmetallischen Verbindungselementen und den Stützelementen stellen in diesem Fall natürliche Sollbruchstellen da, so dass sich die Verbindungselemente bei der radialen Expansion wenigstens teilweise von den Stützelementen lösen und die Stützelemente für die Streckbewegung freigeben können. Aber auch bei einer formschlüssigen Verbindung zwischen Verbindungselementen und Stützelementen können die Stützelemente aus ausreichend weichen Verbindungselementen herausgezogen werden und sich so von den Verbindungselementen lösen. Trotz der groben Ausgestaltung der Verbindungselemente wird die Streckbewegung der Stützelemente daher nicht behindert.

Nichtmetallische Materialien können darüber hinaus ausreichende Elastizität aufweisen, um eine Biegung der Stützprothese in gekrümmten Gefäßen zu gestatten und um einer großen Anzahl von Biegevorgängen des Gefäßes bruchfrei folgen zu können. Nichtmetallische Materialien können ferner biodegradierbar sein und sich im Körper eines Patienten auflösen. Ferner kann daran gedacht werden, das nichtmetallische Material mit Medikamenten zu versetzen, die nach dem Einbringen der Stützprothese an die Gefäßwand abgegeben werden. Derartige Medikamente können insbesondere der Hemmung von Entzündungsreaktionen dienen.

Bei einer bevorzugten Ausführungsform weisen die Stützelemente bei einer radialen Expansion um eine radiale Achse streckbare Bögen auf und die Verbindungselemente sind an den Bögen angebracht. Bei dieser Ausführungsform können sich die Verbindungselemente bei der Streckbewegung der Bögen im Umgebungsbereich der Bögen von der Stützstruktur lösen, so dass die Streckbewegung nicht behindert wird. Im Bereich der Bögen bleiben die Verbindungselemente dagegen in Verbindung mit den Stützelementen und halten die Stützelemente auf Abstand.

Die Stützstruktur selbst kann unterschiedlich ausgebildet sein. In einer Ausführungsform der Stützprothese umfasst die Stützstruktur nebeneinander angeordnete Stützringe, die jeweils in radiale Richtung expandierbar sind und die durch die nichtmetallischen Verbindungselemente zu dem Mantel verbunden sind. Bei einer derartigen Ausgestaltung der Stützstruktur können besonders hohe Kräfte auf die Wand des Gefäßes ausgeübt werden.

Bei einer weiteren Ausgestaltung ist die Stützstruktur frei von Stützelementen, die um den Umfang des Mantels geschlossen umlaufen. Dadurch können in der Stützstruktur keine großräumigen Wirbelströme induziert werden. Die Stützstruktur kann sich daher nicht durch die bei der Anwendung starker Magnetfelder auftretenden Wirbelströme erhitzen. Außerdem kann es bei dem Einsatz von Untersuchungsverfahren wie der nuklearen Magnetresonanz nicht zum Effekt des so genannten Überstrahlens kommen.

Eine Stützstruktur, die frei von geschlossen um den Umfang des Mantels umlaufenden Stützelementen ist, liegt beispielsweise vor, wenn die Stützstruktur von einer Stützhelix gebildet ist. In diesem Fall kann eine große Stützkraft auf die Wand des Gefäßes aufgebracht werden, ohne dass die Gefahr besteht, dass Magnetfelder in der Stützstruktur Wirbelströme induzieren.

Unter den nichtmetallischen Materialien kommen insbesondere Materialien auf der Basis von Chitin oder Chitosan in Frage. Derartige Materialien sind atoxisch, allergische Reaktionen sind nicht bekannt, und die mechanischen Eigenschaften sind variabel einstellbar. Ferner ist Chitin oder Chitosan von der amerikanischen Food and Drug Administration (FDA) als Nahrungszusatzstoff zugelassen. Derartige Materialien sind daher besonders für die Verbindungselemente zwischen den Stützelementen geeignet. Daneben scheinen auch Polymere geeignet zu sein, die ebenfalls atoxisch sind, keine allergischen Reaktionen hervorrufen und eine ausreichende Elastizität aufweisen. Bei einem Ausführungsbeispiel der Stützprothese können die Verbindungselemente streifenförmig ausgebildet sein und in Umfangsrichtung so verlaufen, dass die Verbindungselemente nebeneinander angeordnete Stützeelemente teilweise abdecken. In diesem Fall ist eine besonders hohe Verbindungsfestigkeit zwischen den Stützelementen zu erwarten. Bei einem weiteren Ausführungsbeispiel sind die Verbindungselemente streifenförmig ausgebildet und erstrecken sich in Längsrichtung über eine Vielzahl von Stützelementen. In diesem Fall sind die Anforderungen an die Elastizität der Verbindungselemente besonders niedrig, da die Verbindungselemente bei einer Expansion der Stützelemente nur unwesentlich gestreckt werden müssen.
Daneben ist es möglich, die Verbindungselemente in Zwischenräumen zwischen den Stützelementen anzuordnen. In diesem Fall können die Verbindungselemente durch Eintauchen der vormontierten Stützelemente in eine Lösung, die das für die Verbindungselemente verwendete Material enthält, hergestellt werden. Daneben ist es auch denkbar, die Lösung aufzutröpfeln oder aufzusprühen und die Lösung durch Kapillarkräfte in die Zwischenräume zwischen den Stützelementen einziehen zu lassen.

Weitere Einzelheiten und Vorteile der Erfindung gehen aus der nachfolgenden Beschreibung hervor, in der Ausführungsbeispiele anhand der beigefügten Zeichnung im Einzelnen erläutert werden. Es zeigen:
- Figur 1: eine perspektivische Ansicht eines Stents;
- Figur 2: eine Aufsicht auf zwei nebeneinander angeordnete Stützringe eines Stents, die über ein streifenförmiges Verbindungselement verbunden sind;
- Figur 3: eine Aufsicht auf zwei nebeneinander angeordnete Stützringe, die auf verschiedene Weise durch in Längsrichtung verlaufende streifenförmige Verbindungselemente verbunden werden können;
- Figur 4: eine Aufsicht auf nebeneinander angeordnete Stützringe, mit einem in einem Zwischenraum zwischen den Stützringen angeordneten Verbindungselement;
- Figur 5: eine Aufsicht auf nebeneinander angeordnete Stützringe mit einem weiteren in einem Zwischenraum zwischen den Stützringen angeordneten Verbindungselement;
- Figur 6: eine Aufsicht auf zwei benachbarte Stützringe, deren Zwischenraum vollständig von einem Verbindungselement gefüllt ist;
- Figur 7: eine Aufsicht auf zwei Stützringe, die von einem flächenmäßig ausgebildeten Verbindungselement überdeckt sind; und
- Figur 8: eine Aufsicht auf einen aufgeschnittenen Mantel eines Stents, der eine Stützhelix aufweist.

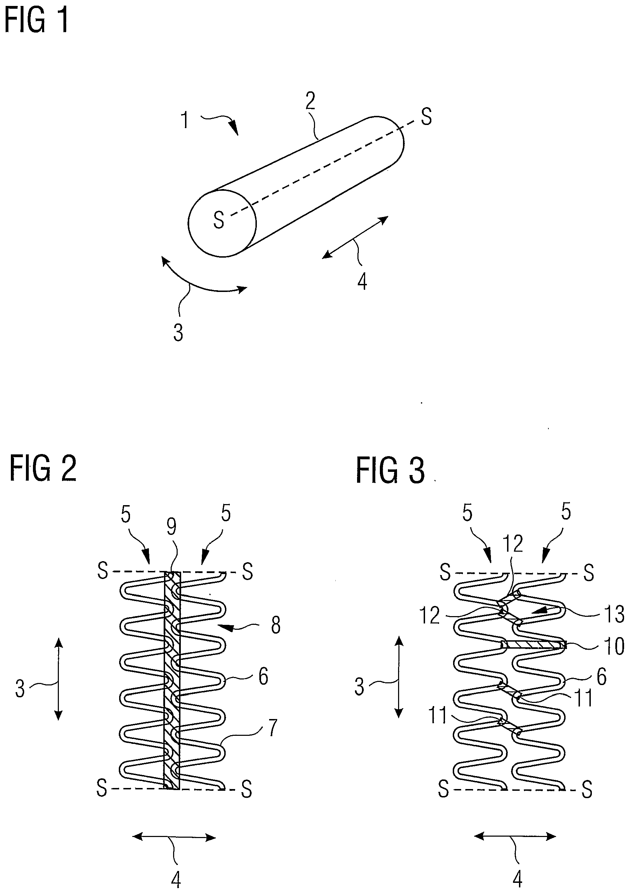

Figur 1 zeigt eine perspektivische Ansicht eines Stents 1, der einen röhrenförmigen Mantel 2 aufweist. Der Mantel 2 weist eine Vielzahl von in eine Umfangsrichtung 3 verlaufenden und in eine Längsrichtung 4 nebeneinander angeordnete Stützringe 5 auf, die in der folgenden Figur 2 bis 7 vergrößert und entlang einer Schnittlinie S-S aufgeschnitten dargestellt sind.

Die Stützringe 5 weisen einen mäanderförmigen, insbesondere wellenförmigen Verlauf auf. Bei dem in den Figuren 2 bis 7 dargestellten Stützringen 5, die einen wellenförmigen Verlauf zeigen, sind Bögen 6 durch gerade verlaufende Stützstreben 7 verbunden. Jeweils ein Bogen 6 und zwei benachbarte Stützstreben 7 bilden eine Schlaufe 8. Aufeinanderfolgende Schlaufen 8 haben dabei jeweils eine Stützstrebe 7 gemeinsam.

Zur Implantation wird der Stent 1 auf einen so genannten Ballonkatheter aufgekrimpt. Der Ballonkatheter wird dann zu der aufzuweitenden Stelle des zu behandelnden Gefäßes gebracht und dort expandiert. Dadurch werden die Stützringe 5 in Umfangsrichtung 3 gestreckt. Insbesondere werden die Bögen 6 im Extremfall so weit um eine radiale Achse aufgebogen, dass der Stützring 5 in einer Ebene verläuft. Typischerweise vergrößert sich der Umfang der Stützringe 5 dabei etwa um das 4,5-fache.

Bei dem in Figur 2 dargestellten Ausführungsbeispiel verlaufen die Mäandermuster der nebeneinander angeordneten Stützringe 5 jeweils mit gleicher Phase. Das bedeutet, dass die sich nach links offene Schlaufen 8 eines ersten Stützrings 5 und nach links offene Schlaufen 8 eines zweiten Stützrings 5 sowie nach rechts offene Schlaufen 8 des ersten Stützrings 5 und nach rechts offene Schlaufen 8 des zweiten Stützrings 5 jeweils gegenüberliegen. Im Bereich der Bögen 6 sind die Stützringe 5 bei dem in Figur 2 dargestellten Ausführungsbeispiel durch einen in Umfangsrichtung 3 verlaufenden, zwischen den Stützringen 5 verlaufenden Verbindungsstreifen 9 verbunden. Für den Verbindungsstreifen 9 wird vorzugsweise ein Material verwendet, das aufgrund seiner elastischen Eigenschaften der Expansion der Stützringe 5 folgen kann. Anderenfalls werden sich die Stützringe 5 während der radialen Expansion von dem Verbindungsstreifen 9 ablösen. Damit der Verbindungsstreifen 9 auch in diesem Fall den Abstand zwischen den Stützringen 5 sicherstellt, muss die radiale Dicke des Stützringes 5 ausreichend groß bemessen sein, so dass die Stützringe 5 auch im gestreckten Zustand von dem ringförmigen Verbindungsstreifen 9 auf Abstand gehalten werden.

In Figur 3 ist ein weiteres Ausführungsbeispiel des Stents 1 dargestellt, bei dem die Stützringe 5 phasengleich nebeneinander angeordnet sind. Anhand Figur 3 sind verschiedenen Möglichkeiten dargestellt, die Stützringe 5 mit Hilfe von längsgerichteten Verbindungselementen zu verbinden. So ist in Figur 3 ein in Längsrichtung 4 verlaufender Verbindungsstreifen 10 dargestellt, der die Bögen 6 zweier nach links offenen Schlaufen 8 verbindet. Bei einer Expansion des Stents 1 braucht der Verbindungsstreifen 10 kaum expandiert zu werden. Bei einer Expansion des Stents wird ein Verbindungsstreifen von der Art des Verbindungsstreifens 10 typischerweise bis auf das 1,5-fache gedehnt. Somit kann für den Verbindungsstreifen 10 verhältnismäßig steifes Material verwendet werden, das die Stützringe 5 auf Abstand hält. Letzteres ist wichtig, wenn der Stent 1 mit Hilfe eines Ballonkatheters eingebracht wird, da ansonsten die Stützringe 5 bei der radialen Expansion aufeinandergeschoben werden.

Daneben ist es möglich, die Bögen 6 benachbarter Schlaufen 8 durch kurze Verbindungsstreifen 11 zu verbinden. Die Verbindungsstreifen 11 können jeweils so angeordnet sein, dass jeweils ein Bogen 6 des einen Stützrings 5 mit einem nächstliegenden Bogen 6 des anderen Stützrings 5 verbunden ist. In diesem Fall verkürzt sich der Stent bei einer Expansion der Stützringe 5 erheblich, wenn nicht Sollbruchstellen in den Verbindungsstreifen 11 vorgesehen sind. Bei einem abgewandelten Ausführungsbeispiel des Stents 1 verbinden kurze Verbindungsstreifen 12 jeweils einen Bogen 6 des einen Stützrings mit zwei verschiedenen Bögen 6 des anderen Stützrings 5. Da bei diesem Ausführungsbeispiel die Verbindungsstreifen 12 das Öffnen einer Schlaufe 13 des zweiten Stützrings 5 verhindern, werden die kurzen Verbindungsstreifen 12 bei einer Expansion der Stützringe 5 zerrissen oder von den Stützringen 5 abgelöst. Vorzugsweise ist daher bei den Verbindungsstreifen 12 eine Sollbruchstelle vorgesehen, an der der Riss der kurzen Verbindungsstreifen 12 erfolgt. Nach der Expansion der Stützringe 5 sind die Stützringe 5 untereinander nicht mehr verbunden. Die Stützringe 5 sind daher in diesem Fall frei bewegbar, so dass dieses Ausführungsbeispiel besonders geeignet für Gefäße erscheint, die häufigen und periodischen Verformungen ausgesetzt sind. Derartige Gefäße sind beispielsweise die Koronargefäße, die sich bei jeder Bewegung des Herzmuskels verformen.

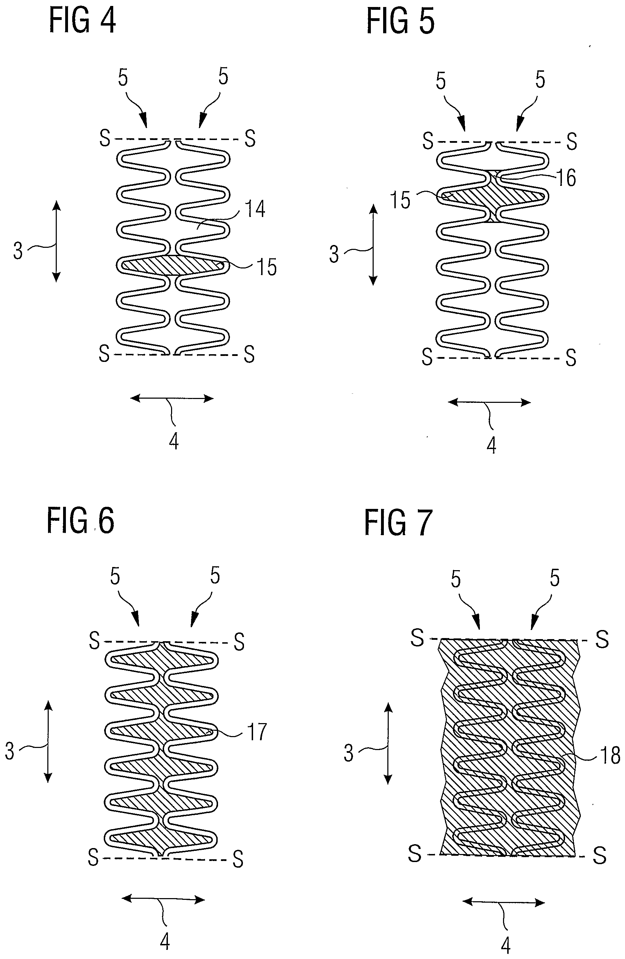

In Figur 4 ist ein weiteres Ausführungsbeispiel dargestellt, bei dem das Mäandermuster von nebeneinander liegenden Stützringen jeweils um 180° in der Phase versetzt ist. Daraus folgt, dass die Bögen 6 von nebeneinander liegenden Stützringen 5 jeweils dicht an dicht liegen. Die Schlaufen 8 nebeneinander liegender Stützringe bilden daher Kammern 14, in der beispielsweise eine Verbindungsschicht 15 von der in Figur 4 dargestellten Art ausgebildet sein kann.
Die Verbindungsschicht 15 braucht nicht notwendigerweise aus einem äußerst elastischen Material gefertigt zu werden. Denn bei einer Expansion der Stützringe 5 wird die Verbindungsschicht 15 entlang den Stützstreben 7 abreißen. Im Bereich der Bögen 6 bleibt die Verbindungsschicht 15 dagegen an den Stützringen 5 haften. Bei einer ausreichenden Steifigkeit der Verbindungsschicht 15 werden die Stützringe 5 auf Abstand gehalten. Die Verbindungsschicht 15 kann besonders einfach hergestellt werden, da die von den nebeneinander liegenden Stützringen 5 geformte Kammer 14 für die Formgebung der Verbindungsschicht 15 herangezogen werden kann. Denn die Kammer 14 begrenzt die seitliche Ausdehnung der Verbindungsschicht 15. So ist es beispielsweise möglich, die Stützringe 5 auf eine Unterlage aufzubringen, und die Kammer 14 mit dem Material der Verbindungsschicht 15 zu füllen.

Dies kann beispielsweise durch Auftropfen einer geeigneten Lösung erfolgen. Dabei wird die Lösung wie in Figur 5 dargestellt durch die Kapillarkräfte auch in Engstellen 16 zwischen dicht nebeneinander liegenden Bögen 6 von nebeneinander angeordneten Stützringen 5 gezogen.

Grundsätzlich ist es denkbar, alle Kammern 14 zwischen den Stützringen 5 durch Verbindungsschichten 17 zu füllen, die sich über den gesamten Umfang der Stützringe 5 erstrecken. Die Verbindungsschichten 17 lassen sich am einfachsten durch Eintauchen des Mantels 2 in eine Lösung des für die Verbindungsschichten 17 verwendeten Materials herstellen.

Figur 7 zeigt schließlich ein Ausführungsbeispiel des Stents 1, bei dem auf den Mantel 2 eine durchgehende Verbindungsschicht 18 aufgebracht worden ist. Dies ist insbesondere dann von Vorteil, wenn das Material für die Verbindungsschicht 18 im gelösten Zustand eine hohe Viskosität aufweist und auf den Mantel 2 des Stents 1 aufgewalzt werden kann. Denkbar ist auch, das Material der Verbindungsschicht 18 in Form einer Röhre auf den Stent 1 aufzuschieben und anschließend aufzuschrumpfen. Daneben ist es auch denkbar, die Verbindungsschicht 18 als Röhre auszubilden, auf deren Außenseite die einzelnen Stützringe 5 aufgeschoben und anschließend fixiert werden.

Es sei angemerkt, dass die Verbindungsschicht 18 auch netzförmig ausgebildet sein kann.

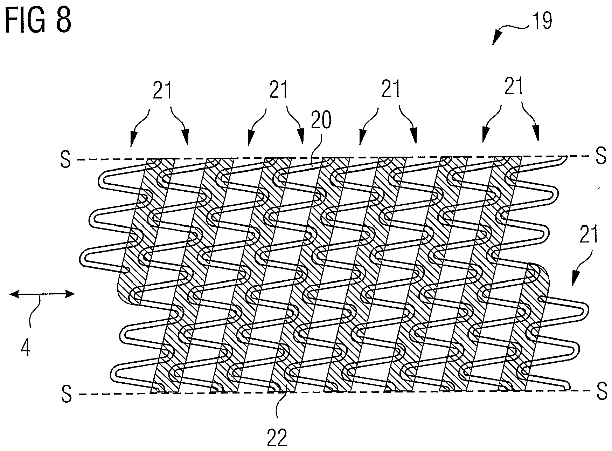

Figur 8 zeigt einen weiteren entlang der Schnittlinie S-S aufgeschnittenen Stent 19, dessen tragende Struktur von einer metallischen Stützhelix 20 gebildet ist. Der Stent 19 ist so dargestellt, wie er entlang einer Schnittlinie S-S aufgeschnitten und auf einer ebenen Fläche flach aufliegend erscheinen würde. In diesem Zustand ist die Stützhelix 20 in Helixabschnitte 21 unterteilt, die jeweils dem Verlauf der Stützhelix 20 bei einem Umlauf um 360° entsprechen. Die Helixabschnitte 21 zeigen einen mäanderförmigen Verlauf und erstrecken sich quer zur Längsrichtung 4 des Stents 19.

Die einzelnen Helixabschnitte 21 können nun entlang der Längsrichtung 4 durch Verbindungselemente von der Art der Verbindungsstreifen 9 bis 12 sowie der Verbindungsflächen 15, 17 und 18 verbunden werden. Bei dem in Figur 8 dargestellten Stent 19 sind die Helixabschnitte 21 beispielsweise durch einen Verbindungsstreifen 22 von der Art des Verbindungsstreifens 9 verbunden.

Der Stent 19 weist zusätzlich zu seiner großen Elastizität den Vorteil auf, dass in der Stützhelix 20 keine großräumigen Wirbelströme induziert werden können. Dadurch stellt der Stent 19 kein Hindernis für die Anwendung von Therapie- oder Diagnoseverfahren dar, die mit starken Magnetfeldern arbeiten. Denn der Stent 19 kann nicht bei starken Magnetfeldern durch das Auftreten von Wirbelströmen erwärmt oder erhitzt werden. Im Rahmen der nuklearen Magnetresonanz werden auch keine Bildartefakte, wie das so genannte Überstrahlen erzeugt. Es dürfte im Gegenteil sogar möglich sein, Bilder vom Inneren des Stent 19 mit den genannten Verfahren zu gewinnen. Dies setzt allerdings einen genügend großen Abstand zwischen den Helixabschnitten 21 voraus.

Ferner kann daran gedacht werden, den Stent 19 mit Kathetern zu platzieren, die eine magnetische Spitze aufweisen, die mit Hilfe von starken magnetischen Feldern navigiert wird.

Die hier beschriebenen Stents 1 und 19 eignen sich insbesondere zum Aufweiten von Koronargefäßen. Da sie ein hohes Maß an Flexibilität aufweisen, können sie auch in stark gekrümmte Gefäße eingebracht werden. Außerdem sind die Stents 1 und 19 dazu in der Lage, häufigen periodischen Bewegungen der Gefäße zu folgen.

Als Material für die Verbindungselemente 9 bis 11 sowie 15, 17, 18 und 22 kommen auf der Basis von Chitin hergestellte Materialien in Frage. Chitin, bei dem es sich um N-acetyl-D-glucos-2-amin handelt, kann auch mit Sklerotin oder dessen Vorstufen versetzt sein. Durch Kochen können die Acetylgruppen von Chitin getrennt werden. Dabei entsteht Chitosan. Es erscheint denkbar, die Verbindungselemente 9 bis 11 sowie 15, 17, 18 und 22 auch auf der Basis von Chitosan herzustellen.

Daneben kommen auch andere Glucosamine enthaltende oder davon abgeleitete Materialien, wie zum Beispiel Zellulosederivate, als Material für die Verbindungselemente 9 bis 11 sowie 15, 17 und 18 in Betracht.

Schließlich ist auch denkbar für die Verbindungselemente 9 bis 11 sowie 15, 17, 18 und 22 Kunststoffe aus der Gruppe der Polymere oder Elastomere zu verwenden, sofern diese biokompatibel sind.

Es sei angemerkt, dass das für die Verbindungselemente 9 bis 11 sowie 15, 17, 18 und 22 verwendeten Materialen auch metallische Komponenten enthalten können. Die metallischen Komponenten können einzelne Partikel, Spuren oder dünne Beschichtungen sein, die keine mechanische Verbindung zwischen den Stützringen 5 oder den Helixabschnitten 21 schaffen.

## Patentansprüche

1. Stützprothese für Gefäße oder intrakorporale Lumina mit einem rohrförmigen, in radiale Richtung expandierbaren Mantel (2), der eine metallische Stützstruktur mit quer zur Längsrichtung (4) nebeneinander liegenden, mäanderförmig verlaufenden Stützelementen (5, 21) aufweist, wobei in Längsrichtung (4) nebeneinander angeordnete Stützelemente (5, 21) durch wenigstens ein sich in Längsrichtung (4) erstreckendes Verbindungselement aus einem elastischen nichtmetallischen Material zu dem röhrenförmigen Mantel (2) verbunden sind,
**dadurch gekennzeichnet, dass** die Bögen (6) der in der Phase versetzten Mäandermuster von nebeneinander liegenden Stützelementen (5, 21) dicht an dicht liegen und die Schlaufen (8) von nebeneinanderliegenden Stützringen Kammern (14) bilden, und dass
das Verbindungselement eine Verbindungsschicht (15, 17) ist, die in einer der von nebeneinander liegenden Stützelementen (5, 21) gebildeten und die Verbindungsschicht in seitliche Richtung begrenzenden Kammern (14) ausgebildet ist.

2. Stützprothese nach Anspruch 1,
**dadurch gekennzeichnet, dass** die Stützelemente (5, 21) durch ein eine Streckbewegung der Stützelemente (5, 21) in Umfangsrichtung (3) zulassendes und die Stützelemente (5, 21) bei der radialen Expansion auf Abstand haltendes Verbindungselement (15, 17) verbunden sind.

3. Stützprothese nach Anspruch 1 oder 2,
**dadurch gekennzeichnet, dass** die Stützelemente (5, 21) bei einer radialen Expansion um eine radiale Achse streckbare Bögen (6) aufweisen und die Verbindungselemente (15, 17) im Bereich der Bögen (6) angebracht sind.

4. Stützprothese nach Anspruch 3,
**dadurch gekennzeichnet, dass** das Verbindungselement (15, 17) bei der radialen Expansion vom Umgebungsbereich der Bögen (6) lösbar an der Stützstruktur angebracht ist.

5. Stützprothese nach einem der Ansprüche 1 bis 4,
**dadurch gekennzeichnet, dass** die Stützstruktur in der Längsrichtung (4) nebeneinander angeordnete Stützringe (5) aufweist, die durch wenigstens ein Verbindungselement (15, 17) aus einem elastischen nichtmetallischen Material zu dem röhrenförmigen Mantel (2) verbunden sind.

6. Stützprothese nach einem der Ansprüche 1 bis 4,
**dadurch gekennzeichnet, dass** die Stützstruktur (20) frei von im Umfangsrichtung (3) geschlossen um den Mantel (2) umlaufenden Stützelementen ist.

7. Stützprothese nach Anspruch 6,
**dadurch gekennzeichnet, dass** die Stützstruktur von einer Stützhelix (20) gebildet ist, deren in Längsrichtung (4) nebeneinander liegenden Helixabschnitte (21) durch wenigstens ein Verbindungselement (15, 17) aus einem elastischen nichtmetallischen Material zu dem röhrenförmigen Mantel (2) verbunden sind.

8. Stützprothese nach einem der Ansprüche 1 bis 7,
**dadurch gekennzeichnet, dass** das nichtmetallische Material auf der Basis von Chitin hergestellt ist.

9. Stützprothese nach Anspruch 8,
**dadurch gekennzeichnet, dass** das nichtmetallische Material auf der Basis von Chitosan hergestellt ist.

10. Stützprothese nach einem der Ansprüche 1 bis 7,
**dadurch gekennzeichnet, dass** das Material ein Polymer ist.

11. Stützprothese nach einem der Ansprüche 1 bis 10,
**dadurch gekennzeichnet, dass** das Verbindungselement eine sich in Engstellen (16) zwischen nebeneinander liegenden Stützelementen (5, 21) erstreckende Verbindungsschicht (15) ist.

## Claims

1. Support prosthesis for vessels or intracorporeal lumens with a tubular casing (2) which can expand in the radial direction, comprising a metallic support structure with support elements (5, 21) extending transversely to the longitudinal direction (4) and arranged next to one another in a meandering pattern, wherein the support elements (5, 21) disposed next to each other in the longitudinal direction (4) are connected to the tubular casing (2) by at least one connecting element which is made of a resilient, non-metallic material and extends in the longitudinal direction (4),
**characterized in that**
the curves (6) of the meandering pattern of the support elements (5, 21) are shifted in phase and are lying close to each other, and the loops (8) of the support rings form chambers (14), and that
the connecting element is a connecting layer (15, 17), which is configured in one of the chambers (14), which are formed by support elements (5, 21) located next to each other and are limiting the lateral extension.

2. Support prosthesis according to claim 1,
**characterized in that**
the support elements (5, 21) are connected by a connecting element (15, 17) which allows the support elements (5, 21) to stretch in the circumferential direction (3) and keeps the support element (5, 21) apart from each other on radial expansion.

3. Support prosthesis according to claim 1 or 2,
**characterized in that**
the support elements (5, 21) comprise curves (6), which can stretch about a radial axis on radial expansion, and that the connecting elements (15, 17) are attached to the regions of the curves (6).

4. Support prosthesis according to claim 3,
**characterized in that**
the connecting element (15, 17)) is attached to the support structure so as to be detachable from the region surrounding the curves (6) on radial expansion.

5. Support prosthesis according to any one of claims 1 to 4,
**characterized in that**
the support structure has support rings (5) which are arranged next to one another in the longitudinal direction (4) and are connected to the tubular casing (2) by at least one connecting element (15, 17) made of a resilient, non-metallic material.

6. Support prosthesis according to any one of claims 1 to 4,
**characterized in that**
the support structure (20) is free from support elements which encircle the casing (2) in a closed manner in the circumferential direction (3).

7. Support prosthesis according to claim 6,
**characterized in that**
the support structure is formed by a support helix (20), of which the helix portions (21) located next to one another in the longitudinal direction (4) are connected to the tubular casing (2) by at least one connecting element (15, 17) made of a resilient, non-metallic material.

8. Support prosthesis according to any one of claims 1 to 7,
**characterized in that**
the non-metallic material is produced based on chitin.

9. Support prosthesis according to claim 8,
**characterized in that**
the non-metallic material is produced based on chitosan.

10. Support prosthesis according to any one of claims 1 to 7,
**characterized in that**
the material is a polymer.

11. Support prosthesis according to any one of claims 1 to 10,
**characterized in that**
the connecting element is a connecting layer (15) extending in narrowings (16) between support elements (5, 21) located next to each other.

## Revendications

1. Prothèse de soutien pour vaisseaux ou lumières intracorporelles avec une enveloppe tubulaire (2) pouvant se dilater dans la direction radiale qui présente une structure de soutien métallique avec des éléments de soutien (5, 21) situés côte à côte transversalement à la direction longitudinale (4), s'étendant en forme de méandre, dans laquelle des éléments de soutien (5, 21) disposés côte à côte dans la direction longitudinale (4) sont reliés par au moins un élément de connexion s'étendant dans la direction longitudinale (4) constitué d'un matériau non métallique élastique à l'enveloppe tubulaire (2),
**caractérisée en ce que**
les arcs (6) du modèle de méandre déphasé d'éléments de soutien (5, 21) situés côte à côte se situent de manière compacte et les boucles (8) d'anneaux de soutien juxtaposés forment des chambres (14), et que l'élément de connexion est une couche de connexion (15, 17) qui est réalisée dans une des chambres (14) formées par des éléments de soutien (5, 21) situés côte à côte et limitant la couche de connexion dans la direction latérale.

2. Prothèse de soutien selon la revendication 1,
**caractérisée en ce que**
les éléments de soutien (5, 21) sont reliés par un élément de connexion (15, 17) autorisant un mouvement d'allongement des éléments de soutien (5, 21) dans la direction périphérique (3) et maintenant les éléments de soutien (5, 21) à l'écart lors de la dilatation radiale.

3. Prothèse de soutien selon la revendication 1 ou 2,
**caractérisée en ce que**
les éléments de soutien (5, 21) présentent des arcs (6) pouvant s'allonger autour d'un axe radial lors d'une dilatation radiale et les éléments de connexion (15, 17) sont appliqués dans la région des arcs (6).

4. Prothèse de soutien selon la revendication 3,
**caractérisée en ce que**
l'élément de connexion (15, 17) est appliqué de manière amovible à la structure de soutien lors de la dilatation radiale de la région environnante des arcs (6).

5. Prothèse de soutien selon une des revendications 1 à 4,
**caractérisée en ce que**
la structure de soutien présente des anneaux de soutien (5) disposés côte à côte dans la direction longitudinale (4) qui sont reliés par au moins un élément de connexion (15, 17) constitué d'un matériau non métallique élastique à l'enveloppe tubulaire (2).

6. Prothèse de soutien selon une des revendications 1 à 4,
**caractérisée en ce que**
la structure de soutien (20) est exempte d'éléments de soutien circulant dans la direction périphérique (3) de manière fermée autour de l'enveloppe (2).

7. Prothèse de soutien selon la revendication 6,
**caractérisée en ce que**
la structure de soutien est formée d'une hélice de soutien (20) dont les sections d'hélice (21) situées côte à côte dans la direction longitudinale (4) sont reliées par au moins un élément de connexion (15, 17) constitué d'un matériau non métallique élastique à l'enveloppe tubulaire (2).

8. Prothèse de soutien selon une des revendications 1 à 7,
**caractérisée en ce que**
le matériau non métallique est fabriqué à base de chitine.

9. Prothèse de soutien selon la revendication 8,
**caractérisée en ce que**
le matériau non métallique est fabriqué à base de chitosane.

10. Prothèse de soutien selon une des revendications 1 à 7,
**caractérisée en ce que**
le matériau est un polymère.

11. Prothèse de soutien selon une des revendications 1 à 10,
**caractérisée en ce que**
l'élément de connexion est une couche de connexion (15) s'étendant en goulots (16) entre des éléments de soutien (5, 21) situés côte à côte.
